# EUROPÄISCHE PATENTSCHRIFT

(11) **EP 0 873 108 B1**
(45) Veröffentlichungstag und Bekanntmachung des Hinweises auf die Patenterteilung: **20.04.2005**
(21) Anmeldenummer: 97911177.0
(22) Anmeldetag: 04.10.1997
(51) Int. Cl.: A61K 7/06

(54) **VERWENDUNG VON BLÜTENWACHS IN FLÜSSIGEN HAARBEHANDLUNGSMITTELN**
USE OF FLOWER WAX IN LIQUID HAIR-TREATMENT AGENTS
UTILISATION DE CIRE FLORALE DANS DES AGENTS DE SOINS CAPILLAIRES LIQUIDES

(30) Priorität: 13.10.1996 DE 19641992
(43) Veröffentlichungstag der Anmeldung: 28.10.1998
(73) Patentinhaber: Wella Aktiengesellschaft, 64274 Darmstadt (DE)
(72) Erfinder: KRIPP, Thomas, D-64407 Fränkisch-Crumbach (DE); KRAUSE, Beate, D-65795 Hattersheim (DE); LANG, Günther, D-64354 Reinheim (DE); MEURER, Wolfgang, D-68623 Lampertheim (DE); TOECHE-MITTLER, Ingrid, D-64285 Darmstadt (DE)
(86) Internationale Anmeldenummer: PCT/EP1997/005459
(87) Internationale Veröffentlichungsnummer: WO 1998/016188

(56) Entgegenhaltungen:
- US-A- 5 460 808

## Beschreibung

Es ist bekannt, daß natürliche pflanzliche Wachse wie Carnaubawachs oder Candelillawachs in kosmetischen Zubereitungen bereits vielfältig Anwendung finden. Aus der internationalen Patentanmeldung WO 93/17 083 ist außerdem die Verwendung von Apfelwachs für die Herstellung von kosmetischen Mitteln zur Pflege und Reinigung von Haut und Haaren bereits bekannt. Die wertvollen Eigenschaften dieser bereits genauer untersuchten natürlichen pflanzlichen Wachse hat inzwischen auch dazu geführt, daß moderne Extraktionsverfahren entwickelt worden sind, mit denen pflanzliche Wachse in guten Ausbeuten und frei von störenden Begleitstoffen isoliert werden können.

Während die aus pflanzlichen Blüten isolierbaren ätherischen Öle insbesondere zur Herstellung von Duftstoffen und Parfüms starkes Interesse auf sich gezogen haben, sind die aus pflanzlichen Blüten isolierbaren Wachse bisher nur wenig beachtet worden. Nähere Untersuchungen haben jedoch gezeigt, daß die aus Pflanzenblüten isolierbaren Wachse eine Reihe von wertvollen Eigenschaften in sich vereinen, die in Zukunft eine zunehmende kommerzielle Bedeutung derartiger Wachse erwarten lassen.

Wie alle natürlichen Pflanzenwachse zeichnen sich auch die Blütenwachse durch hydrophobe Eigenschaften, durch Schmelzbarkeit, Emulgierbarkeit und biologische Abbaubarkeit aus. Diese Eigenschaften sind nun speziell bei Blütenwachsen mit weiteren anwendungstechnischen Parametern kombiniert, die für den Einsatz in Haarbehandlungsmitteln gute Voraussetzungen bilden.

Aus der japanischen Offenlegungsschrift 21 608 von 1984 sind Hautbehandlungsmittel (Creme, Lippenstift) und ein fester Haarstift mit einem Gehalt an Jasminblütenwachs beschrieben. Flüssige Haarbehandlungsmittel werden dort nicht erwähnt.

Die US-A 5 460 808 beschreibt eine flüssige Emulsion, die neben weiteren Bestandteilen auch aus Pflanzenblüten isoliertes Wachs enthält. Das aus Blüten isolierte Wachs wird in Mascara eingesetzt, um die Geschmeidigkeit, Flexibilität und Weichheit des durch die Mascara erzeugten Films zu erhöhen.

Gegenstand der Erfindung ist die Verwendung eines aus Pflanzenblüten isolierten Wachses in einem flüssigen Haarbehandlungsmittel gemäß Anspruch 1. Besonders das Wachs aus den Blüten des Jasmins (Jasminum officinale), aus der Mimose (Mimosa pudica), aus der Narzisse (Narzissus spec.), der Bitterorange (Ponciurus trifoliata) und der Wildkamille (Matricaria chamomilla) sind bereits näher untersucht worden und haben bemerkenswerte und wertvolle Eigenschaften gezeigt.

Auffällig ist bei derartigen Zubereitungen ein hohes Aufziehvermögen auf das Haar. Schon bei minimalen Konzentrationen ist bereits eine starke Pflegewirkung auf das Haar zu beobachten. Daneben fallen eine ausgeprägte Glanzgebung bei haarkosmetischen Zubereitungen und eine deutliche Fixierung eingearbeiteter Duftstoffe auf. Ein Blütenwachs enthaltendes flüssiges Haarbehandlungsmittel zeichnet sich außerdem durch Hautverträglichkeit, ein gutes Feuchthaltevermögen und eine rückfettende Wirkung z. B. in Haarwaschlotionen aus. Diese Wirkungen werden erreicht, wenn das Blütenwachs in dem flüssigen Haarbehandlungsmittel in einer Menge von 0,001 bis 20 Gewichtsprozent, vorzugsweise in einer Menge von 0,01 bis 12 Gewichtsprozent, besonders bevorzugt in einer Menge von 0,03 bis 2 Gewichtsprozent, enthalten ist. Die Blütenwachse finden deshalb besonders Anwendung in Haarkuren, Shampoos, Stylingpräparaten, Haarbehandlungsmittel in Schaumform, Haarfärbemitteln, Haarsprays und Haarlotionen. Dabei werden die Blütenwachse in Kombination mit allen für flüssige Haarbehandlungsmittel üblichen Kosmetikrohstoffen zur Anwendung gebracht, jedoch mit Ausnahme von stark oxidierenden Substanzen wie Wasserstoffperoxid. Die flüssigen Haarbehandlungsmittel können insbesondere als Lösung, W/O- oder O/W-Emulsion oder als flüssiges Gel oder Schaum vorliegen.

Der Nachweis des für die Schutz- und Pflegewirkung von Blütenwachsen auf Haare charakteristischen Aufziehvermögens läßt sich mit der Röntgen-Photoelektronen-Spektroskopie (XPS) eindeutig führen. Es handelt sich hierbei um eine oberflächensensitive Meßtechnik, die es ermöglicht, die Elementzusammensetzung sowie die Bindungszustände der Elemente in den äußersten Molekülschichten (Informationstiefe wenige Nanometer) zu bestimmen. Für oberflächenanalytische Fragestellungen aus dem Bereich der Korrosion, der Metallurgie, der Katalyse, der Adhäsion, der Mikroelektronik und der Polymertechnologie hat die Röntgen-Photoelektronen-Spektroskopie bereits seit längerem Anwendung gefunden. Sie hat sich jedoch auch zur Untersuchung der komplexen Struktur von Fasern, zum Beispiel der Wolle, bewährt, so daß diese Materialien jetzt standardmäßig mit der XPS vermessen werden können. Diese Methode eignet sich aber auch für eine Oberflächenbewertung der mit Pflegemitteln behandelten Haaroberfläche, weil deren Elementarzusammensetzung bis zu einer Eindringtiefe von etwa 10 nm bestimmt werden kann. Das Ergebnis einer mit der XPS-Methode durchgeführten Messung einer Haaroberfläche ist die Elementarzusammensetzung in Atom % Kohlenstoff (C), Atom % Sauerstoff (O), Atom % Stickstoff (N) und Atom % Schwefel (S) der Haaroberfläche in einer Schichtdicke von etwa 10 nm. Für das Haarkeratin, das ein Protein ist, sind insbesondere die Meßergebnisse bezüglich des Gehalts der Oberfläche in Atom % an Stickstoff und Schwefel charakteristisch.

Wird die Haaroberfläche mit einem Pflegemittel beschichtet, das in seiner Zusammensetzung im wesentlichen die Elemente Kohlenstoff und Sauerstoff aufweist, jedoch nur geringe Anteile oder gar keinen Stickstoff oder Schwefel hat, dann müssen abhängig von der Güte des Aufziehvermögens des Pflegemittels die Anteile (in Atom %) des Schwefels und des Stickstoffes an der gemessenen Oberfläche zurückgehen, da mit der XPS nur die äußeren 10 nm Schichtdicke des Haares erfaßt werden. Der Gehalt der gemessenen, mit Haarpflegemittel behandelten Oberfläche an Schwefel und Stickstoff (in Atom %) ist daher ein Maß für das Aufziehvermögen des Haarpflegemittels. Je geringer der Anteil des Schwefels und des Stickstoffs (in Atom %) an der Elementarzusammensetzung der behandelten Haaroberfläche ist, um so gleichmäßiger und dichter ist der durch das Pflegemittel auf dieser Oberfläche hinterlassene Film und um so größer ist also das Aufziehvermögen des Haarpflegemittels.

Die Anwendung der Röntgen-Photoelektronen-Spektroskopie auf die Untersuchung des Aufziehvermögens von Haarpflegemitteln wurde nun in der Weise durchgeführt, daß eine handelsübliche Basispflegerezeptur durch Austausch des in ihr enthaltenen Fettalkohols gegen je 1% Apfel-, Jasmin- oder Mimosenwachs abgewandelt und das Aufziehvermögen der so geänderten Rezeptur auf Haare gemessen wurde. Dabei ließ man die Basispflegerezeptur und die durch Blütenwachszusatz abgeänderten Rezepturen jeweils 5 Minuten auf Haare einwirken, die dann gründlich ausgewaschen wurden. Als Maß für die Ausbildung eines Schutzfilms wurde die Verminderung der detektierten proteintypischen Elemente "Stickstoff' und "Schwefel" durch die Pflegerezepturen gewertet. Die Apfelwachs enthaltende Vergleichsrezeptur, die bereits früher entwickelt worden war, wurde dabei als eine besonders hochwertige Testrezeptur zum Vergleich eingesetzt, denn die damit erreichte Schutzwirkung war bisher von keiner anderen der getesteten Kosmetiklipide auch nur annäherungsweise erreicht worden. Überraschenderweise zeigte sich jedoch, daß das Aufziehvermögen von Jasminwachs noch um mehr als 10% über dem Aufziehvermögen des Apfelwachses liegt. Bei der XPS-Messung zeigte sich nämlich, daß das mit dem Apfelwachsprodukt gepflegte Haar gegenüber dem mit Jasminwachs behandelten noch 19% mehr Stickstoff und 11% mehr Schwefel auf seiner Oberfläche hatte.

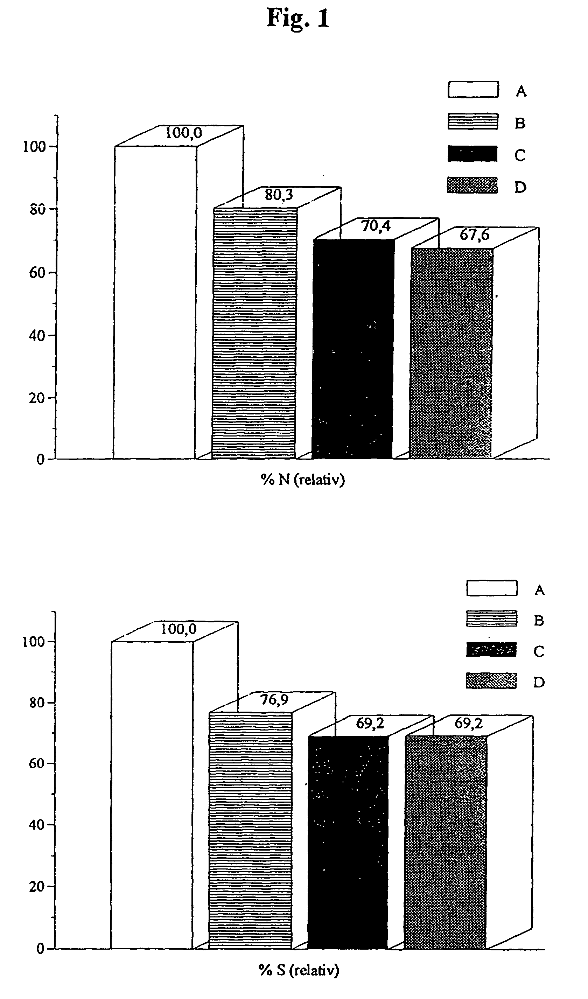

In Fig. 1 ist die Elementarzusammensetzung der Haaroberfläche in Atom % für Stickstoff und Schwefel quantitativ dargestellt, wobei für beide Elemente die jeweilige Atomkonzentration bei Anwendung der Basisrezeptur A auf 100% festgelegt wurde. Enthält diese Basisrezeptur (A) nur 1% Apfelwachs (B), Mimosenwachs (C) oder Jasminwachs (D), nimmt die Menge der oberflächenexponierten Haarproteine gegenüber der Basisrezeptur (A) um über 30% ab. Die Basisrezeptur (A) hat dabei folgende Zusammensetzung:

**Tab.: 1**

| Zusammensetzung der Basisrezeptur | | | | |
|---|---|---|---|---|
| **Rohstoff** | **Rezeptur A in %** | **Rezeptur B in %** | **Rezeptur C in %** | **Rezeptur D in %** |
| Cetylalkohol | 2,0 | 2,0 | 2,0 | 2,0 |
| Paraffinöl | 2,0 | 1,0 | 1,0 | 1,0 |
| Apfelwachs | . | 1,0 | . | . |
| Mimosenwachs | . | . | 1,0 | . |
| Jasminwachs | . | . | . | 1,0 |
| Sorbitanstearat | 1,0 | 1,0 | 1,0 | 1,0 |
| Lanolinöl, Isopropylpalmitat, Rizinusöl | 1,0 | 1,0 | 1,0 | 1,0 |
| Glycerin | 2,0 | 2,0 | 2,0 | 2,0 |
| Laurylakoholpolyethylenglykolether (20EO) | 1,0 | 1,0 | 1,0 | 1,0 |
| Cetyltrimethylammoniumchlorid -Lösung 25%ig | 2,0 | 2,0 | 2,0 | 2,0 |
| Wasser | 89,0 | 89,0 | 89,0 | 89,0 |

Weiterhin wurde gefunden, daß mit dem Austausch von Carnauba- oder Candelillawachs in der Basisrezeptur durch jedes der oben genannten Blütenwachse eine Verbesserung der Kämmbarkeit erreicht wird. Besonders ausgeprägt ist diese Wirkung im Falle des Bitterorangenblütenwachses. Beim Narzissenwachs zeigt sich zwar die geringste kämmbarkeitsverbessernde Wirkung von allen untersuchten Blütenwachsen, jedoch erhielt dafür das Haar Volumen und eine deutlich erhöhte Frisurstabilität. Beim Kamillenwachs ergab sich eine Erhöhung des Glanzes.

Die erfindungsgemäß verwendeten Blütenwachse werden in ungereinigter Form als Abfallrohstoff bei der Parfümöl/Aromengewinnung erhalten. Die Rohwachse bedürfen jedoch noch einer Aufreinigung, um sie von Resten des Chlorophylls, von Duftstoffen und polaren Verunreinigungen zu befreien. Diese Aufreinigung erfolgt nach an sich bekannten physikalischen Adsorptionsmethoden, die beispielsweise in der internationalen Patentanmeldung W 93/17 083 für die Reinigung von Apfelswachs beschrieben sind. Zweckmäßigerweise wird das Rohwachs zunächst in heißem Hexan aufgelöst, dann in Gegenwart von Bleicherde unter Rückflußtemperatur gerührt und anschließend ohne Rühren und Heizen stehen gelassen, bis sich die mit den Verunreinigungen beladene Bleicherde abgesetzt hat. Der noch warme Ansatz wird anschließend filtriert und das Filtrat durch Destillation vom Lösungsmittel befreit. Die physikalischen Daten der erfindungsgemäß eingesetzten Blütenwachse sind Tabelle 3 zu entnehmen.

**Tab.: 3**

| Kennzahlen der Blütenwachse | | | | | |
|---|---|---|---|---|---|
| **Blütenwachse** | **Jasmin** | **Mimose** | **Bitterorange** | **Wildkamille** | **Narzisse** |
| Tropfunkt [°C] | 62,4 | 69,3 | 59,9 | 67,0 | 63,4 |
| Jodzahl [g J/100g] | 37,9 | 26,1 | 70 | 71 | 66 |
| Peroxidzahl | <1,0 | 3,9 | 32 | 41 | 29 |
| Säurezahl [mg KOH/g] | 2,0 | 13,0 | 9 | 20 | 7 |
| Verseifungszahl [mg KOH/g] | 68,0 | 59,0 | 96 | 99 | 51 |
| Esterzahl [VZ-SZ] | 66,0 | 46,0 | 87 | 79 | 44 |

Die folgenden Beispiele zeigen die Einsatzmöglichkeiten von Blütenwachsen in flüssigen Haarbehandlungsmitteln.

### Beispiel 1 - Haarspray zur Anwendung bei feuchtem Klima

| **Rohstoff** | **%** | **%** | **%** | **%** | **%** | **%** |
|---|---|---|---|---|---|---|
| Vinylacetat/Crotonsäure-Copolymer | 2,00 | 2,00 | 2,00 | 2,00 | 2,00 | 2,00 |
| | | | | | | |
| 2-Amino-2-methyl-1-propanol | 0,16 | 0,16 | 0,16 | 0,16 | 0,16 | 0,16 |
| | | | | | | |
| Äthanol, wasserfrei | 37,84 | 37,84 | 37,84 | 37,84 | 37,84 | 37,84 |
| | | | | | | |
| Parfümöl | 0,10 | 0,10 | 0,10 | 0,10 | 0,10 | 0,10 |
| | | | | | | |
| Carnaubawachs | 0,05 | | | | | |
| Jasminwachs | | 0,05 | | | | |
| Mimosenwachs | | | 0,05 | | | |
| Bitterorangenblütenwachs | | | | 0,05 | | |
| Narzissenwachs | | | | | 0,05 | |
| Wildkamillenwachs | | | | | | 0,05 |
| | | | | | | |
| Propan/Butan ad | 100,00 | 100,00 | 100,00 | 100,00 | 100,00 | 100,00 |

Der Ersatz des Carnaubawachses durch ein Blütenwachs führt zu einer Verbesserung der Kämmbarkeit und einer hohen Frisurstabilität.

### Beispiel 2 - Cremeshampoos

| **Rohstoff** | **%** | **%** | **%** | **%** | **%** | **%** |
|---|---|---|---|---|---|---|
| Fettalkoholsulfate: Natriumlauryl-,-myristyl-, -cetyl-,-stearylsulfat; Laureth-10 | 35,0 | 35,0 | 35,0 | 35,0 | 35,0 | 35,0 |
| Stearinsäure | 9,0 | 9,0 | 9,0 | 9,0 | 9,0 | 9,0 |
| | | | | | | |
| Carnaubawachs | 0,5 | | | | | |
| Jasminwachs | | 0,5 | | | | |
| Mimosenwachs | | | 0,5 | | | |
| Bitterorangenblütenwachs | | | | 0,5 | | |
| Narzissenwachs | | | | | 0,5 | |
| Wildkamillenwachs | | | | | | 0,5 |
| | | | | | | |
| NaCl | 3,0 | 3,0 | 3,0 | 3,0 | 3,0 | 3,0 |
| | | | | | | |
| Triäthanolamin, rein | 4,0 | 4,0 | 4,0 | 4,0 | 4,0 | 4,0 |
| | | | | | | |
| 1,2-Dibrom-2,4-dicyanobutamin-2-phenoxyethanol | 0,1 | 0,1 | 0,1 | 0,1 | 0,1 | 0,1 |
| Wasser ad | 100,0 | 100,0 | 100,0 | 100,0 | 100,0 | 100,0 |

Die erfindungsgemäßen Rezepturen führen zu einer deutlich feststellbaren Erhöhung des Glanzes und einer Verbesserung der Kämmbarkeit des Haares

### Beispiel 3 - Intensivhaarkur

| **Rohstoff** | **%** | **%** | **%** | **%** | **%** | **%** |
|---|---|---|---|---|---|---|
| Glycerinmonostearat, neutral | 6,0 | 6,0 | 6,0 | 6,0 | 6,0 | 6,0 |
| | | | | | | |
| Lanolinalkoxylat | 2,0 | 2,0 | 2,0 | 2,0 | 2,0 | 2,0 |
| | | | | | | |
| Carnaubawachs | 1,0 | | | | | |
| Jasminwachs | | 1,0 | | | | |
| Mimosenwachs | | | 1,0 | | | |
| Bitterorangenblütenwachs | | | | 1,0 | | |
| Narzissenwachs | | | | | 1,0 | |
| Wildkamillenwachs | | | | | | 1,0 |
| | | | | | | |
| Cetylalkohol | 2,0 | 2,0 | 2,0 | 2,0 | 2,0 | 2,0 |
| | | | | | | |
| Gemisch aus Lanolinalkohol und Paraffinöl | 1,0 | 1,0 | 1,0 | 1,0 | 1,0 | 1,0 |
| | | | | | | |
| Tris-(oligooxyethyl)-alkylammoniumphosphat | 1,5 | 1,5 | 1,5 | 1,5 | 1,5 | 1,5 |
| | | | | | | |
| Hydroxyethylcellulose | 20,0 | 20,0 | 20,0 | 20,0 | 20,0 | 20,0 |
| | | | | | | |
| Citronensäure | 0,1 | 0,1 | 0,1 | 0,1 | 0,1 | 0,1 |
| | | | | | | |
| Sorbinsäure | 0,2 | 0,2 | 0,2 | 0,2 | 0,2 | 0,2 |
| | | | | | | |
| Wasser ad | 100,0 | 100,0 | 100,0 | 100,0 | 100,0 | 100,0 |

Das erfindungsgemäße Intensivhaarkurmittel läßt sich problemlos nach der Anwendung aus dem Haar spülen, ergibt eine gute Naß- und Trockenkämmbarkeit und verleiht dem Haar einen guten Griff und ein gepflegtes Aussehen.

### Beispiel 4 - Schaumkonditionierer mit Festiger

| **Rohstoff** | **%** | **%** | **%** | **%** | **%** | **%** |
|---|---|---|---|---|---|---|
| PVP/Vinylimidazoliniummethochlorid-Copolymer | 5,00 | 5,00 | 5,00 | 5,00 | 5,00 | 5,00 |
| | | | | | | |
| PVP/PVA-Copolymer | 1,00 | 1,00 | 1,00 | 1,00 | 1,00 | 1,00 |
| | | | | | | |
| Polyoxyethylen-12-cetylstearylalkohol | 0,15 | 0,15 | 0,15 | 0,15 | 0,15 | 0,15 |
| | | | | | | |
| Parfümöl | 0,10 | 0,10 | 0,10 | 0,10 | 0,10 | 0,10 |
| | | | | | | |
| Candelillawachs | 0,05 | | | | | |
| Jasminwachs | | 0,05 | | | | |
| Mimosenwachs | | | 0,05 | | | |
| Bitterorangenblü- | | | | | | |
| tenwachs | | | | 0,05 | | |
| Narzissenwachs | | | | | 0,05 | |
| Wildkamillenwachs | | | | | | 0,05 |
| | | | | | | |
| Wasser ad | 100,00 | 100,00 | 100,00 | 100,00 | 100,00 | 100,00 |
| | | | | | | |
| Propan/Butan 40/60 | 10,00 | 10,00 | 10,00 | 10,00 | 10,00 | 10,00 |

Der Schaumkonditionierer ergibt einen seidigen Schaum und verbessert die Kämmbarkeit und den Griff des damit gewaschenen Haares.

### Bezugszeichenliste zu Fig.1:

### Abdeckung der Haaroberfläche durch Blütenwachse

- A: Basisrezeptur
- B: Basisrezeptur mit 1% Apfelwachs
- C: Basisrezeptur mit 1% Mimosenwachs
- D: Basisrezeptur mit 1% Jasminwachs

## Patentansprüche

1. Verwendung eines aus Pflanzenblüten isolierten Wachses in einem flüssigen Haarbehandlungsmittel in einer Menge von 0,001 bis 20 Gewichtsprozent umfassend die Pflege, die Glanzgebung, die Fixierung eingearbeiteter Duftstoffe, die Hautverträglichkeit, das Feuchthaltevermögen und/oder die rückfettende Wirkung.

2. Verwendung nach Ansprüchen 1, **dadurch gekennzeichnet, dass** die Menge des aus Pflanzenblüten isolierten Wachses 0,01 bis 12 Gewichtsprozent beträgt.

3. Verwendung nach Ansprüchen 1 und 2, **dadurch gekennzeichnet, dass** das aus Pflanzenblüten isolierte Wachs aus Blüten des Jasmins, der Mimose, der Narzisse, der Bitterorange oder der Wildkamille isoliert worden ist.

4. Verwendung nach Ansprüchen 1 bis 3, **dadurch gekennzeichnet, dass** die aus Pflanzenblüten isolierten Wachse in Haarkuren, Shampoos, Stylingpräparaten, Haarbehandlungsmitteln in Schaumform, Haarfärbemitteln, Haarsprays und Haarlotionen Anwendung finden.

5. Verwendung nach Ansprüchen 1 bis 4, **dadurch gekennzeichnet, dass** die Haarbehandlungsmittel als Lösung, als eine Öl/Wasser- oder eine Wasser/Öl-Emulsion, als ein flüssiges Gel oder als flüssiger Schaum vorliegt.

## Claims

1. Use of a wax isolated from plant flowers in a liquid hair-treatment agent in an amount of from 0.001 to 20 per cent by weight comprising the care, the imparting of shine, the fixing of incorporated fragrances, the skin compatibility, the moisturization and/or the refatting effect.

2. Use according to Claim 1, **characterized in that** the amount of the wax isolated from plant flowers is 0.01 to 12 per cent by weight.

3. Use according to Claims 1 and 2, **characterized in that** the plant isolated from plant flowers has been isolated from the flowers of jasmine, of mimosa, of narcissus, of bitter orange or of wild camomile.

4. Use according to Claims 1 to 3, **characterized in that** the waxes isolated from plant flowers are used in hair treatments, shampoos, styling preparations, hair-treatment compositions in foam form, hair colorants, hairsprays and hair lotions.

5. Use according to Claims 1 to 4, **characterized in that** the hair-treatment agent is in the form of a solution, an oil/water emulsion or a water/oil emulsion, a liquid gel or liquid foam.

## Revendications

1. Utilisation d'une cire isolée à partir de fleurs, dans une composition liquide de traitement capillaire, en une quantité de 0,001 à 20 % en poids, comprenant le soin, le lustrage, le fixage de parfums incorporés, la tolérance par la peau, le pouvoir de rétention d'humidité et/ou l'effet de regraissage.

2. Utilisation selon la revendication 1, **caractérisée en ce que** la quantité de la cire isolée à partir de fleurs va de 0,01 à 12 % en poids.

3. Utilisation selon les revendications 1 et 2, **caractérisée en ce que** la cire isolée à partir de fleurs a été isolée à partir de fleurs du jasmin, du mimosa, du narcisse, de l'orangé amer ou de la camomille sauvage.

4. Utilisation selon les revendications 1 à 3, **caractérisée en ce que** les cires isolées à partir de fleurs sont utilisées dans des lotions de traitement capillaire, des shampooings, des préparations de coiffage, des compositions de traitement capillaire sous forme de mousse, des compositions de teinture pour cheveux, des laques pour cheveux et des lotions capillaires.

5. Utilisation selon les revendications 1 à 4, **caractérisée en ce que** la composition de traitement capillaire se trouve sous forme de solution, sous forme d'une émulsion huile/eau ou d'une émulsion eau/huile, sous forme d'un gel liquide ou sous forme de mousse liquide.
